# EUROPEAN PATENT APPLICATION

(11) **EP 0 669 136 A1**
(43) Date of publication of application: **30.08.1995**
(21) Application number: 94500097.4
(22) Date of filing: 01.06.1994
(51) Int. Cl.: A61L 9/03, A61L 9/14

(54) **Odorized steam emitting device applicable to domestic and industrial cleaning machines**

(30) Priority: 23.02.1994 ES 9400493
(71) Applicant: HISPAINOX S.A., E-46018 Valencia (ES)
(72) Inventor: Cervera Palomares, D. Vicente, E-46026 Valencia (ES)
(74) Representative: De la Fuente Fernandez, Dionisio

(57) **Abstract**

The present specification refers to an odorized steam emitting device, applicable to domestic and industrial cleaning machines, the evident purpose of which is to emit, after being adapted to a connection located on the own cleaning machine, adequately odorized steam outside, via a nozzle, in accordance with the characteristics of the odorizing fluid located outside an auxiliary tank containing same, connected to the nozzle, said device having an electric current socket plugged to the connection base, and a nozzle to receive steam from a boiler located inside the cleaning machine, the odorizing fluid being capable of being replaced by a perfuming, disinfectant, relaxing, therapeutic fluid, and so on.

## Description

### SUMMARY OF THE INVENTION

The present specification refers to a Model of Utility related to an odorized steam emitting device, applicable to domestic and industrial cleaning machines, the evident purpose of which is to emit, after being adapted to a connection located on the own cleaning machine, adequately odorized steam outside, via a nozzle, in accordance with the characteristics of the odorizing fluid located outside an auxiliary tank containing same, connected to the nozzle, said device having an electric current socket plugged to the connection base, and a nozzle to receive steam from a boiler located inside the cleaning machine, the odorizing fluid being capable of being replaced by a perfuming, disinfectant, relaxing, therapeutic fluid, and so on.

### FIELD OF THE INVENTION

This invention applies to the industry devoted to the manufacture of domestic and industrial cleaning machines, generating steam.

### SUMMARY OF THE INVENTION

The odorized steam emitting device, applicable to domestic and industrial cleaning machines as proposed by the invention, constitutes per se an evident novelty within the field to which it is incorporated, since starting of same, it is possible to have at user's disposal, after being incorporated in a cleaning machine, fitted with a boiler, an auxiliary device capable of being adapted to the connection base of hoses and other ancilliary elements, this device acting in an independent way according to the existence, inside a casing constituting the device, of a thermostat which gradually turns off the current of the electrical resistors located inside, so preventing subsequent damages.

In a most specific manner, the odorized steam emitting device, applicable to domestic and industrial cleaning machines is constituted starting from a casing, chassis or frame, made of a rigid plastic material, fully hollow inside, having, at its back side, fixing links on a fitting base located on the machine, having an emerging nozzle suitable for being hermetically connected with the steam outlet through which the steam generated inside the boiler located on the machine escapes, while at the end or opposite side of this back arrangement, there are corresponding terminals to be connected to the plugging base, which is situated on the same portion of the machine.

The nozzle through which the steam is directed is connected to an inner channel finishing, at the opposite side, in a complementary part fixed to it by conventional means. This complementary part has a fixing surface for the opposite nozzle, thru which the directed steam stream arrives at this receptacle.

In said hollow part, having an outlet acting as emitting - nozzle, there is a portion at its lower side, which is adequately shaped, which allows for placing and adapting a tank containing a perfuming, disinfectant, odorizing, relaxing, therapeutic or similar fluid, counting on the corresponding sealing element.

A wick, emerging from the tank or perfume or scent bottle, enters the casing acting as a nozzle, which is directed toward the interior of an inner portion in a body integral with it, adopting a cylindrical shape, this body having a central hole, and the wick is surrounded by a resistance at this area.

At the lower side or nozzle base, a complementary resistance is provided, which is covered by water coming from the steam condensation in its path from a steam generating apparatus to the outlet of the emitting nozzle. The function of said resistance in cooperation with the resistance located at the upper side, is to cause the mentioned water evaporation, this water acting, at the same time, as a coolant for the own resistance.

The resistance is electrically fed by means of a connection taking energy from a base located at the back side.

Such as it has been previously said, within the main body of the large casing of the device, a thermostat has been provided, said thermostat acting, in a harmonious collaboration, with the resistance incorporated in the front end forming the nozzle, so impeding an overcharge of same, and acting as a cutting element at the right moment, avoiding the resistances to be overheated, and subsequent damages in the casings incorporating the resistances, such as it may happen in the resistance located at the inner area owing to the lack of water because this resistance has caused the evaporation of its surrounding water.

Also, the thermostat will act when it detects a lack of fluid within the tank containing the odorizing or similar fluid, upon detecting that the wick transferring the fluid until the nozzle has not the required humidity degree.

The casing or main body of the device counts on an anchoring element at the fitting base on the machine, this anchoring element being fitted with a push-button for being released.

### BRIEF DESCRIPTION OF THE DRAWINGS

In order to complement this description and to aid to a better understanding of the features of the invention, the accompanying drawings, which are a part of this specification, show in an illustrative but non limitative sense, the following:
Figure 1 is a side elevational view of an odorized steam emitting device, applicable to domestic and industrial cleaning machines, coupled to a support base located on a side of a cleaning machine fitted inside with a corresponding boiler.
Figure 2 shows a side elevational view of the object of the invention, showing at the lower portion of the nozzle area, a tank containing an odorized or similar fluid.
Figure 3 shows a plan view taken on the upper side of the object illustrated in Fig. 2.
Figure 4 shows a front elevational view taken on the back side corresponding to a fitting base to the machine of the object illustrated in Figs. 2 and 3.
Figure 5 shows a side elevational view, duly sectioned, of the object of the invention, in a similar view to that illustrated in Fig. 2, incorporating a marginal view of the fitting or supporting base incorporated in the cleaning machine.

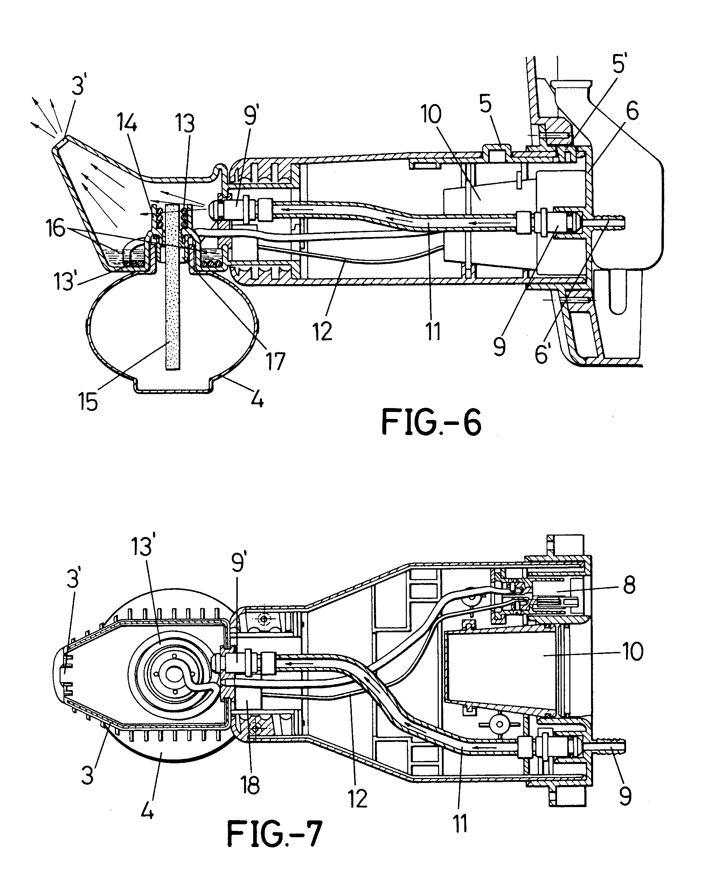

Figure 6 shows a side elevational view having a configuration which is similar to that illustrated in Fig. 5, showing also the device fixed to the supporting base.
Figure 7 shows, lastly, a plan view, duly sectioned, of the object of the invention illustrated in previous Figs.

### DETAILED DESCRIPTION OF THE INVENTION

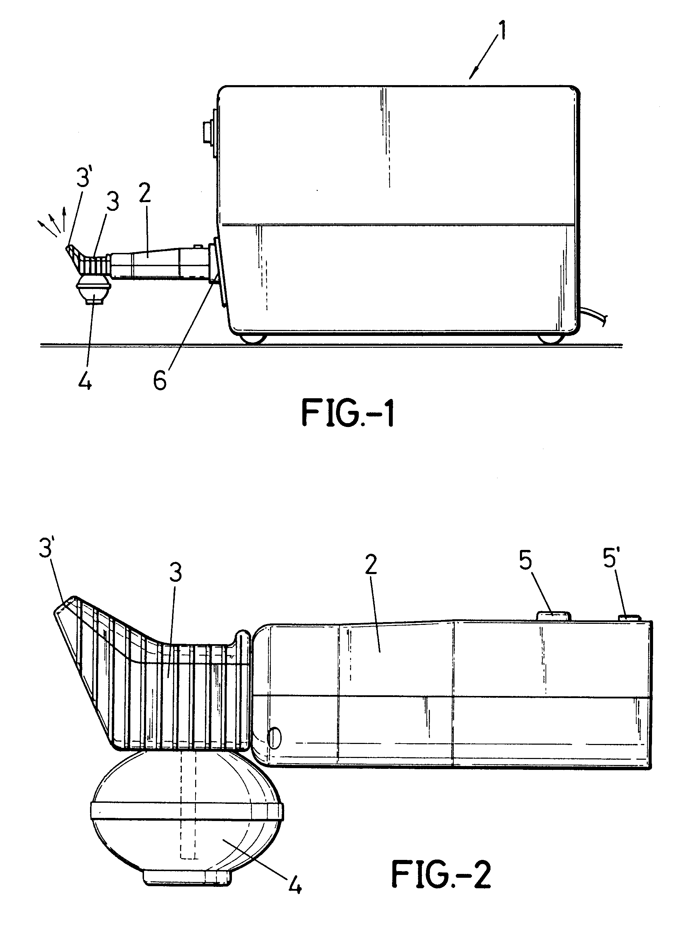

From Figure 1, it can be noted the manner in which the odorized steam emitting device, applicable to domestic and industrial cleaning machines (1) is constituted starting from a body (2), particularly elongated and hollow, made of a rigid plastic material, which has, at one of its ends, a part (3), also made of plastic material, having at its end an emerging deformation at the upper portion of which there is a hole (3'), the part (3) acting in collaboration with the hole (3') as a nozzle; said part (3) having, at its lower side, a body or container (4) containing an odorized or similar fluid, which, in collaboration with the steam emitted by a boiler of the cleaning machines (1), projects outside through the hole (3') similar to a microdropper.

Following Figure 2, it can be noted that the device has, at the upper side of the casing or main body (2), a push-botton (5), the function of which is to release the fixing link (5') on the fixing base (6) located on a side of the cleaning machine (1).

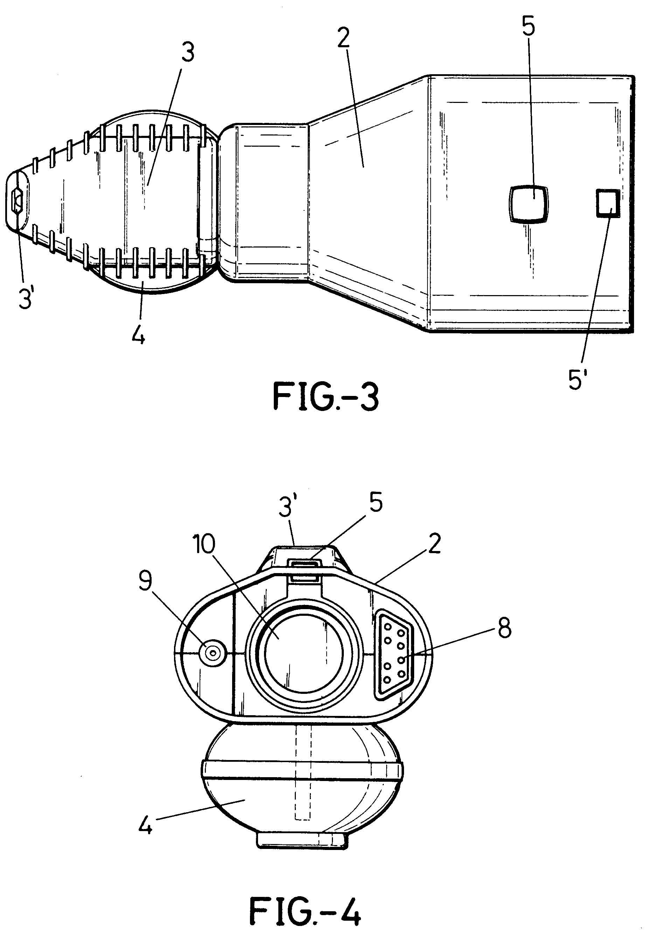

Figure 3 shows a plan view of the device, in which it can be clearly seen that it is constituted by a main casing (2), having a nozzle (3) having a hole (3') , and on the lower side of the fitting portion there is a container with an odorizing fluid (4).

Likewise, on the upper side of the main or central casing (2). there is a releasing push-botton (5) of the fixing link (5') on the base (6) of machine (1

Following Figure 4, it can be noted how the casing (2) has an ample back mouth having an electrical connection (8) where appropiate fitting terminals to the connecting side located at the fixing base (6) of machine (1) are located; the connecting nozzle (9), through which the steam emitted by the machine (1) boiler will pass; and a hermetic central area (10) to cut the intake side located on the supporting base (6) of the machine (1), in case that the machine was fitted with the intake function, said function not being necessary for operating this odorized steam emitting device.

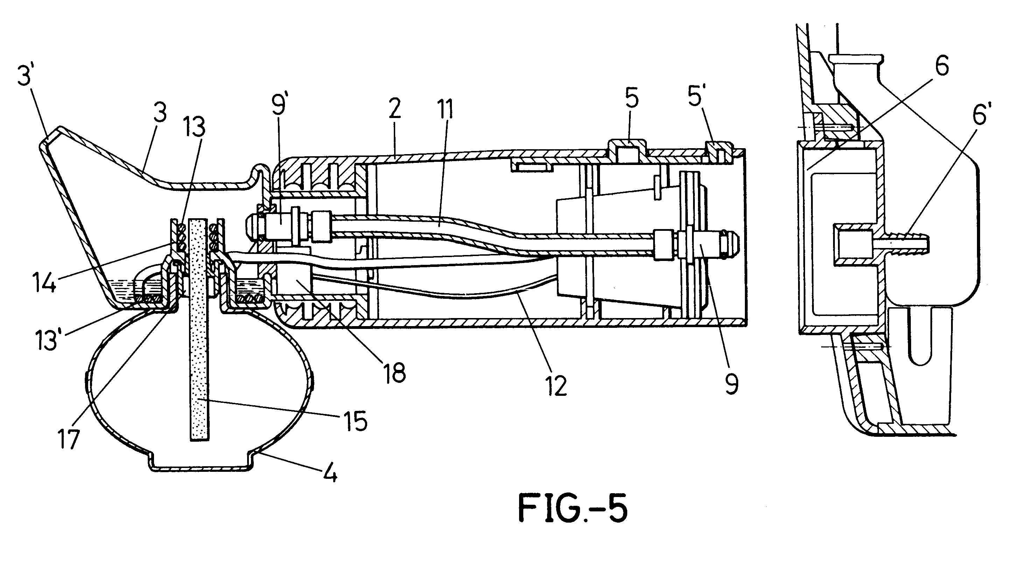

In Figure 5, it is noted again the device duly sectioned, showing that inside the casing (2) there is located the fixing part mounted on the connection base (6), by means a link (5'), which can be released from its locking position by means of a push-botton (5), having inside a blind area (10) impeding the machine (1) intake to act. It is convenient to repeat that the mentioned intake device is not necessary if the machine performs an intake function, since said device only works starting from the utilization of the steam emitted by the boiler, which is located in the machine, having, at the same time, a connecting - nozzle (9) placed on an inner channel terminal (11), and connected with the steam outlet (6') on the base (6), while the other end of the channel (11) is fitted with a nozzle (9') through which the steam will be expelled toward the main nozzle (3), the inside of which is hollow and it is coupled to the container (4) fitted with a wick (15) emerging toward the inner portion of the body (3) in a hollow cylindrical area (14) which is internally surrounded by a resustance (13) which is fed by means of a cable )12) connected with terminals (8) which are coupled to the fixing base (6) of machine (1).

The container (4) has a shutter (17) for the wick (15), the container being coupled to a hollow cylindrical deformation on the lower side of the body (3). On this lower side of the body (3), there is a complementary resistance (13') surrounding the adapting deformation of the container (4), this resistance being fed, like the resistance (13) by the cable (12) connected to the terminals (8).

Lastly, the invention counts on a thermostat (18).

The present device operates after fixing the body (3), by means of a retainer (5), in the base (6). So, it is possible to connect, on the one hand, the nozzle (9), adapted to the channel (11), to the steam outlet (6') of the machine (1), while, on the other hand, the electric terminals (8) are connected to the corresponding area of the base (6), the machine intake being plugged by the blind - area (10), provided that the machine relies on an intake function. When the machine (1) has reached the adequate temperature, the - steam will be expelled from the machine (1) through the outlet - (6'), passing via the nozzle (9) to, afterwards, pass through the conduct or channel (11) up to the second nozzle (9'), and upon being expelled inside the hollow body (3), it will be drained outside through the hole (3'), its motion in search of an outlet carrying with it, this outlet being the hole (3'), the steams emitted by the wick (15), hastened by the resistance (13) and, in turn, impelled by the resistance (13'). So the fluid is deposited on the lower area of the hollow body (13) due to the steam condensation caused by its cooling at the outlet, until arriving at the nozzle (9'), and then it evapores and is carried by the steam flow until the outlet hole (3').

It is to be pointed out that the neck of the container (4) fits in the deformation existing on the lower side of the hollow part (3), reference with (16), acting as a retaining link of the own container (4).

If the termostat (18) detects an overheating by lack of water inside the body (3), owing to any reason, it will automatically cut off the electric energy directed toward the resistances (13) and (13'), and the operation of the device which is the subject of the invention will automatically stop.

It ia not considered necessary to extend more this description for an expert in the art to understand the scope of the invention and the advantages derived from it.

The materials, shape, size and arrangement of the components are open to variation, provided that it does not imply any alteration to the essence of the invention.

The terms under which this specification has been described should be always be taken in an ample and non limitative sense.

## Claims

1. An odorized steam emitting device applicable to domestic and industrial cleaning machines. of those constituted starting from a casing made of a rigid plastic material, fitted internally with adequated ribs and stiffening deformations, having a mouth at the back side, being internally hollow and markedly elongated, the device being able to optionally replace an odorized fluid by a perfuming, relaxing, therapeuticc, disinfectant, etc. fluid, characterized in that one of the ends of the hollow body has adequate fixing means for a complementary hollow part, fitted with an angular emerging deformation at the upper end of which there is a hole, while the lower side has a cylindrical recess in - which the neck of a receiver containing odorizing or similar fluids is fixed, with a wick which emerges through the inner portion of the hollow body in which it is fixed, fitted with a shutter and being surrounded the end of the wick by an electrical resistance, a second complementary resistance being provided on the lower plant area of the main body fixing the container, and surrounding the - inner mouth of the container fixing body; this second complementary resistance being fed by electrical current via a cable which is connected to a plugging base placed on the back side of the main body.

2. An odorized steam emitting device applicable to domestic and industrial cleaning machines, according to claim 1, characterized in that in the opposite side to that fitted on the plugging base, there is a steam inlet nozzle connected to a steam outlet - placed on the machine, specifically on the connecting base, the main body of the device being held by a link which is displaceable by a push-button mounted on the base of the machine.

3. An odorized steam emitting device applicable to domestic and industrial cleaning machines, according to any of the preceding claims, characterized in that the nozzle through which the steam generated by the machine enters, is connected to a channel having, at the opposite end, a second diffusing nozzle through which the steam generated by the machine passes into a chamber located in the extreme hollow body, within which are placed the resistances and the emerging portion of the wick.

4. An odorized steam emitting device applicable to domestic and industrial cleaning machines, according to any of the preceding claims, characterized in that within the body (2) there is located a thermostat cutting the current passing to the resistance when detecting overheating.

5. An odorized steam emitting device applicable to domestic and industrial cleaning machines, according to any of the preceding claims, characterized in that the resistance situated on the lower portion of the body fitted with a hole through which the odorized steeam emerges outside, acts as an evaporation resistance of fluids located in the lower portion of this part.
